## Europäisches Patentamt
## European Patent Office
## Office européen des brevets

(19)

(11) Publication number: **0 013 131**
A2

(12)

# EUROPEAN PATENT APPLICATION

(21) Application number: **79302969.5**

(22) Date of filing: **19.12.79**

(51) Int. Cl.³: **A 61 K 9/24**

(30) Priority: **27.12.78 GB 4995678**

(43) Date of publication of application:
**09.07.80 Bulletin 80/14**

(84) Designated Contracting States:
**AT BE CH DE FR GB IT LU NL SE**

(71) Applicant: **Mundipharma A.G.**
St. Alban-Vorstadt 94 Postfach
CH-4006 Basel(CH)

(72) Inventor: **Rhodes, Alan**
2, Seaview Terrace
Cove Bay, Aberdeen ABI 4NL(GB)

(72) Inventor: **Leslie, Stewart Thomas**
9, Airyhall Place
Aberdeen(GB)

(74) Representative: **Stagg, Diana Christine et al,**
ELKINGTON & FIFE High Holborn House 52-54 High
Holborn
London WC1V 6SH(GB)

(54) Pharmaceutical composition in solid dosage form, and process for its production.

(57) A pharmaceutical composition in solid dosage form which comprises a core including a first active ingredient and a film coating, characterised in that the film coating contains a second active ingredient.

In a preferred embodiment of the pharmaceutical compositions according to the invention, the core contains an iron source, in particular ferrous glycine sulphate, incorporated in a controlled release matrix, and is coated with a film coating containing a vitamin, for example folic acid.

The pharmaceutical composition according to the invention is preferably produced by preparing a tablet core containing an active ingredient, then preparing a film coating solution or suspension containing a second active ingredient, and spraying the solution or suspension onto the core.

EP 0 013 131 A2

Croydon Printing Company Ltd

COMPLETE DOCUMENT

This invention relates to a pharmaceutical composition in solid dosage form in which active ingredients are incorporated, and to a process for its production.

It is known to prepare dosage forms in which two or more active ingredients are incorporated in a single solid dosage form. There are, however, problems involved in obtaining a solid dosage form wherein the maximum therapeutic efficiency for all of the active ingredients is capable of being realised.

Problems may arise, for example, because of chemical incompatibilities between different active ingredients, or where differences exist in half-lives of elimination.

If a controlled release system is used the maximum therapeutic efficiency cannot in general be realised where the system contains two or more active ingredients with different half-lives of elimination.

One method which has been used in the past to overcome these disadvantages of solid dosage forms containing two or more active ingredients, has been to formulate a bi- or multi- layer tablet, each layer of which contains the required amount of one active ingredient, contained where appropriate in a controlled release system. The manufacture of these bi-layer or multi-layer tablets is difficult and time-consuming. An object of the present invention is to provide a solid dosage form in which the above disadvantages are reduced or substantially obviated.

This invention therefore relates to a pharmaceutical composition in solid dosage form which comprises a core including a first active ingredient and a film coating, characterised in that the film coating contains a second active ingredient.

In principle, the pharmaceutical compositions according to the invention may contain any active ingredients, the choice of active ingredients depending on the desired therapeutic effect.

In a preferred embodiment of the pharmaceutical composition according to the invention, the two active ingredients have different half lives of elimination, the active ingredient of the core requiring incorporation within a controlled release system due to its particular half-life of elimination and being so incorporated while the other ingredient is incorporated within the film coat.

The pharmaceutical compositions according to the invention are particularly suitable in iron and vitamin therapy, the treatment of asthma and bronchitis and in treatment of migraine.

For use in iron and vitamin therapy, the pharmaceutical compositions according to the invention suitably comprise a core containing a suitable iron source, such as ferrous glycine sulphate, ferrous sulphate, ferrous succinate, ferrous fumarate, or ferrous gluconate, incorporated in a controlled release matrix in order to reduce the risk of gastro-intestinal disturbances, the core being film-coated with a coating containing the vitamin to be administered for example, folic acid, riboflavin, thiamine or pyridoxine hydrochloride.

For the treatment of asthma and bronchitis, the compositions according to the invention suitably contain a core containing a bronchodilator, in particular aminophylline or theophylline, incorporated in a controlled release matrix, and film coated with a coating containing, for example, a tranquiliser such as hydroxyzine, chlordiazepoxide or chlorpromazine hydrochloride or, in particular for the treatment of asthma where instantaneous bronchodilation is required, a potentiator such as isoprenaline, isoetharine or protokylol.

For the treatment of migraine, the compositions according to the invention suitably contain a core containing a stimulant such as caffeine, incorporated in a controlled release matrix, and coated with a film coating containing an anti-migraine agent such as ergotamine.

The active ingredient in the film coating may be one which is active in one biological environment and the active ingredient in the core may be one which is active in another biological environment but is rendered inactive in the first.

In the above described iron-containing tablets, the vitamin in the coating is absorbed in the stomach and the iron compound absorbed in the stomach and intestine. In the tablets containing a bronchodilator and a tranquiliser, the tranquiliser will be primarily absorbed first through instantaneous release and the bronchodilator second over a predetermined period of time; where the tablet contains a potentiator the patient can allow the tablet to remain in the buccal cavity to take advantage of the potentiating effect of the potentiator.

Particularly advantageous solid dosage forms according to the invention are those in which the first active ingredient is incorporated in a controlled release system in which the matrix comprises a higher aliphatic alcohol and a hydrated water soluble hydroxyalkyl cellulose. Such matrixes and their production are described and claimed in UK Patent No. 1,405,088, the disclosure of which is incorporated herein by reference.

In preparing the dosage forms according to the invention the following procedure may conveniently be adapted.

A tablet core containing an active ingredient incorporated at the desired concentration is prepared

by known methods. The core containing the active ingredient may for example be prepared by a method as described in UK Patent Specification No. 1,405,088. A film coating solution is then prepared according to a standard film coating formulation, and the second active ingredient is dissolved or suspended in this solution at a suitable concentration. The solution or suspension is then sprayed on the core using standard film coating procedures and apparatus, until the desired quantity of second active ingredient has been deposited on the cores. Under normal production conditions, this procedure can be completed within thirty to forty minutes. If desired, a sealing coat may be applied by the film coating process before and also after the coating containing the active ingredient has been applied.

Film coating is to be distinguished from the older procedure known as sugar coating which is very difficult to apply in practice.

The invention will now be further described with reference to the following Examples.

Example 1

A solid dosage form comprising iron and folic acid for administration to patients suffering from anaemia, and as a prophylactic against folic acid deficiency was prepared as follows:

A core containing iron in the form of ferrous glycine sulphate incorporated in a controlled release matrix according to UK Patent No. 1,405,088 was prepared by the following procedure.

The procedure is for the production of 100,000 tablets containing 100 mg elemental iron per tablet, ferrous sulphate (27.20 kg), glycine (13.40 kg) and hydroxyethyl cellulose (Natrosol 250) (1.5 kg ) were dry-blended until a uniform mix was obtained. Water q.s.

was added and the mixture blended until a uniform granulated mix was obtained.

Reaction between the ferrous sulphate and the glycine was allowed to proceed at a temperature of 70°C for a period of five hours until a hard, crude granulate mass was obtained.

The granular mass so formed was passed through a wet granulator and fluid-bed dried at 60°C for 30 minutes.

The crude granules were passed through a dry granulator (16 mesh, British standard, particle size) and dried again at 50°C for 15 minutes. Molten cetyl alcohol (5.0 kg) was added to the dry granular mass and mixing was continued until a uniform mass was obtained. The mass obtained is passed through a dry granulator (16 mesh) and finally lubricated in a cone blender with talc (0.80 kg) and magnesium stearate (0.40 kg) and compressed into tablet cores. (Punch size 13/32" deep concave).

The tablet cores were then coated with a preliminary sealing coat, containing per litre:-

| | | |
|---|---|---|
| Methocel 15 cps | 32.3 | g |
| Ethocel 10 cps | 8.075 | g |
| Diethyl phthalate | 4.75 | mls |
| Methanol | 332.50 | mls |
| Colourant q.s. | | |
| Methylene chloride | to 1 litre. | |

The sealed cores were then further coated with a coating solution containing per litre:-

| | | |
|---|---|---|
| Folic acid | 5.00 | g |
| Polyethylene glycol (PEG) 300 | 50.00 | g |
| Isopropanol | 300.00 | ml |
| Hydroxypropyl methyl cellulose 15 cps | 34.00 | g |
| Methylene chloride | to 1000 ml | |

The process is finally completed using a secondary sealing coat of the same composition as the first but with a different colourant.

## Example 2

Tablet cores containing iron in the form of ferrous glycine sulphate were prepared by the process described in Example 1.

The tablet cores were then coated with an aqueous film coating solution containing, for 3.0 litres:

| | | |
|---|---|---|
| Folic acid B.P. | 7.50 | g |
| Sodium bicarbonate | q.s to pH 7 - 8 | |
| Polyethylene glycol 400 | 75.00 | ml |
| Hydroxypropylmethyl cellulose 15 cps | 102.00 | g |
| Water | to 3.00 | litres |

The sealed cores were then further coated with a secondary sealing coat using an aqueous film coating solution having a similar composition to the first, containing no folic acid but containing a colourant.

Claims:

1. A pharmaceutical composition in solid dosage form, which comprises a core including a first active ingredient, and a film coating characterised in that the film coating contains a second active ingredient.

2. A composition as claimed in claim 1 characterised in that the two active ingredients have different half lives of elimination, the active ingredient which requires incorporation within a controlled release system due to its particular half-life of elimination being so incorporated, and the other active ingredient being incorporated within the film coat.

3. A composition as claimed in claim 2 characterised in that the controlled release system is a matrix which comprises a higher aliphatic alcohol and a hydrated water soluble hydroxyalkyl cellulose.

4. A composition as claimed in any of claims 1 to 3, characterised in that the core contains an iron source incorporated in a controlled release matrix, and is coated with a film-coating containing a vitamin.

5. A composition as claimed in claim 4 characterised in that the iron source is ferrous glycine sulphate and the vitamin is folic acid.

6. A composition as claimed in any of claims 1 to 3 characterised in that the core contains a bronchodilator and the coating contains a tranquiliser or a potentiator.

7. A composition as claimed in any of claims 1 to 3 characterised in that the core contains a stimulant and the coating contains an anti-migraine agent.

8. A process for the preparation of a pharmaceutical composition as claimed in any of claims 1 to 7 characterised in that a tablet core containing an active ingredient is prepared, a film coating solution or suspension containing a second active ingredient is then prepared and the solution or suspension is sprayed on to the core.

9.    A pharmaceutical composition as claimed in any of claims 1 to 7 characterised in that it is prepared by a process as claimed in claim 8.